(19) Europäisches Patentamt
European Patent Office
Office européen des brevets

(11) EP 0 823 939 B1

(12) **EUROPÄISCHE PATENTSCHRIFT**

(45) Veröffentlichungstag und Bekanntmachung des Hinweises auf die Patenterteilung:
**19.01.2000 Patentblatt 2000/03**

(21) Anmeldenummer: **96912030.2**

(22) Anmeldetag: **23.04.1996**

(51) Int Cl.[7]: **C11D 3/39**, C07C 251/30

(86) Internationale Anmeldenummer:
**PCT/EP96/01685**

(87) Internationale Veröffentlichungsnummer:
**WO 96/34937 (07.11.1996 Gazette 1996/49)**

(54) **N-BENZYLIMINIUMSALZE ALS BLEICHKATALYSATOREN**

N-BENZYLIMINIUM SALTS USED AS BLEACH CATALYSTS

SELS DE N-BENZYLIMINIUM UTILISES EN TANT QUE CATALYSEURS DE BLANCHIMENT

(84) Benannte Vertragsstaaten:
**DE ES FR GB IT NL**

(30) Priorität: **03.05.1995 DE 19515730**

(43) Veröffentlichungstag der Anmeldung:
**18.02.1998 Patentblatt 1998/08**

(73) Patentinhaber: **BASF AKTIENGESELLSCHAFT
67056 Ludwigshafen (DE)**

(72) Erfinder:
- **WEHLAGE, Thomas
D-67346 Speyer (DE)**
- **BOECKH, Dieter
D-67117 Limburgerhof (DE)**
- **OFTRING, Alfred
D-67098 Bad Dürkheim (DE)**

(56) Entgegenhaltungen:
**US-A- 4 210 551**
**US-A- 5 360 568**
**US-A- 5 360 569**

- **CHEMICAL ABSTRACTS, vol. 109, no. 17, 24.Oktober 1988 Columbus, Ohio, US; abstract no. 149330, G. SCHLÜTER ET AL.: "Isoquino (1,2-a)(2)benzazepines" XP002012409 & LIEBIGS ANN. CHEM., 1988, Seiten 833-837, XP000601502**
- **CHEMICAL ABSTRACTS, vol. 88, no. 15, 10.April 1978 Columbus, Ohio, US; abstract no. 105189, C.L. DEYRUP ET AL.: "An intramolecular Cycloaddition" XP002012410 & TETRAHEDRON LETTERS, Bd. 39, 1977, Seiten 3437-3440,**
- **CHEMICAL ABSTRACTS, vol. 98, no. 23, 6.Juni 1983 Columbus, Ohio, US; abstract no. 198489, KAMETANI ET AL.: "A facile total synthesis of (+-)-cherylline via an aziridinium intermediate" XP002012411 & J. CHEM. SOC. , PERKIN TRANS. I, 1982, Seiten 2935-2937, XP000601504**
- **CHEMICAL ABSTRACTS, vol. 106, no. 11, 16.März 1987 Columbus, Ohio, US; abstract no. 083641, A. PAWDA ET AL.: " Use of N-((Trimethylsilyl)methyl)amino Ethers as Capped Azomethine Ylide Equivalents" XP002012412 & J. ORG. CHEM., Bd. 52, 1987, Seiten 235-244, XP000601501**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 823 939 B1

Printed by Jouve, 75001 PARIS (FR)

## Beschreibung

Die vorliegende Erfindung betrifft die Verwendung von N-Benzyliminiumsalzen mit Substituenten einer bestimmten Mindestgröße am Iminiumstickstoff als Bleichkatalysatoren, insbesondere in Textilwaschmittel-Bleichsystemen, welche Peroxyverbindungen enthalten. Weiterhin betrifft die Erfindung Wasch-, Reinigungs- und Bleichmittelformulierungen, insbesondere entsprechende Textilwaschmittelformulierungen, die diese N-Benzyliminiumsalze als Bleichkatalysatoren enthalten. Da ein Teil dieser N-Benzyliminiumsalze neue Stoffe darstellen, betrifft die Erfindung ebenfalls diese neuen Stoffe.

Mit Bleichkatalysatoren eröffnet sich eine neue Technologie der Bleiche. Es sind aufgrund der katalytischen Wirkung deutlich weniger Einsatzstoffe notwendig als zuvor bei den Bleichaktivatoren.

In der US-A 5 360 568 (1) und der US-A 5 360 569 (2) werden quartäre Iminiumsalze als Bleichkatalysatoren in Textilwaschmitteln beschrieben. Als bevorzugt werden dabei Dihydroisochinolinium-Verbindungen mit dem Strukturelement

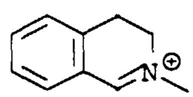

herausgestellt, am Rande werden jedoch auch N-Benzyliminium-Verbindungen erwähnt, z.B.:

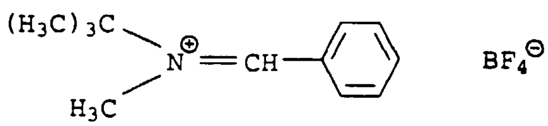
(II)

und

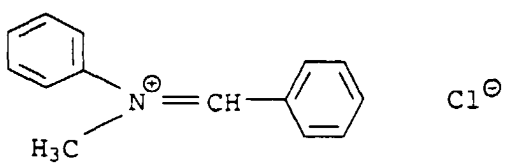
(III)

Diese aus dem Stand der Technik bekannten Verbindungen weisen jedoch noch keine ausreichend gute Bleichwirkung in Waschmittelformulierungen auf.

Aufgabe der vorliegenden Erfindung war es daher, effektivere Bleichkatalysatoren bereitzustellen.

Demgemäß wurde die Verwendung von N-Benzyliminiumsalzen der allgemeinen Formel I

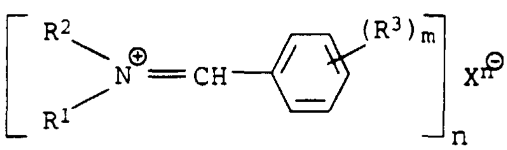
(I)

in der

$R^1$ und $R^2$ unabhängig voneinander Wasserstoff, $C_1$- bis $C_{30}$-Alkyl, $C_2$- bis $C_{30}$-Alkenyl, $C_5$- bis $C_{18}$-Cycloalkyl, $C_7$- bis $C_{18}$-Aralkyl oder -Heteroaralkyl oder $C_6$- bis $C_{18}$-Aryl oder -Heteroaryl bedeuten, wobei aliphatische Gruppierungen zusätzlich durch ein bis fünf Hydroxylgruppen, $C_1$- bis $C_4$-Alkoxygruppen, Aminogruppen, $C_1$- bis $C_4$-Alkylaminogruppen, Di-$C_1$- bis $C_4$-alkylaminogruppen, Chloratome, Bromatome, Nitrogrup-

pen, Cyanogruppen, $C_1$- bis $C_4$-Thioalkylgruppen, $C_1$- bis $C_4$-Sulfoalkylgruppen, Carboxylgruppen, Sulfogruppen, Carboxy-$C_1$- bis $C_4$-alkylgruppen, Carboxyamidgruppen oder Phenyl-, Tolyl- oder Benzylreste funktionalisiert, wobei aromatische, cycloaliphatische und heterocyclische Struktureinheiten ebenfalls durch die genannten Reste substituiert sein können, oder durch ein bis acht nicht benachbarte Sauerstoffatome, Aminogruppen, $C_1$- bis $C_4$-Alkylaminogruppen oder Carbonylgruppen unterbrochen sein können,

mit der Maßgabe, daß wenigstens einer der Reste $R^1$ oder $R^2$ mindestens 5 C-Atome im Falle einer aliphatischen Struktur von $R^1$ bzw. $R^2$ oder mindestens 7 C-Atome im Falle des Vorliegens eines cyclischen Strukturelementes in $R^1$ bzw. $R^2$ aufweist,

$R^3$ die unter $R^1$ bzw. $R^2$ aufgeführten Bedeutungen oder Hydroxyl, Mercapto, $C_1$- bis $C_4$-Alkoxy, Amino, $C_1$- bis $C_4$-Alkylamino, Di-$C_1$- bis $C_4$-alkylamino, Chlor, Brom, Nitro, Cyano, $C_1$- bis $C_4$-Thioalkyl, $C_1$- bis $C_4$-Sulfoalkyl, Formyl, Carboxyl, Sulfo, Sulfato, Carboxy-$C_1$- bis $C_4$-alkyl, Carboxamid, Phenyloxy, Tolyloxy oder Benzyloxy bezeichnet,

m für die Zahl 1, 2 oder 3 steht und

$X^{n\ominus}$ ein oxidationsstabiles Anion bezeichnet, wobei

n die Wertigkeit des Anions X bedeutet,

als Bleichkatalysatoren gefunden.

Bei den N-Benzyliminiumsalzen I muß wenigstens einer der Reste $R^1$ oder $R^2$ eine bestimmte Mindestgröße aufweisen, damit ein optimales Hydrophilie-Hydrophobie-Verhältnis der polaren und unpolaren Molekülteile von I eingestellt werden kann. Dabei können bei langkettigen oder voluminösen Kohlenwasserstoffgruppen für $R^1$ bzw. $R^2$ polare funktionelle Gruppe der Modifizierung der hydrophoben Eigenschaften des Restes dienen.

Die Mindestgröße für $R^1$ bzw. $R^2$ von 5 bzw. 7 C-Atomen wird in den nachfolgend beschriebenen bevorzugten Ausführungsformen durch optimale Bereiche für die Restegröße ergänzt.

Als lineare oder verzweigte Alkylreste, die als $C_1$- bis $C_{30}$-Alkyl-, $C_1$- bis $C_4$-Alkyl-, $C_5$- bis $C_{22}$-Alkyl- und $C_8$- bis $C_{18}$-Alkylreste angesprochen sind, kommen insbesondere in Betracht: Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, n-Hexyl, n-Heptyl, n-Octyl, 2-Ethylhexyl, n-Nonyl, iso-Nonyl, n-Decyl, n-Undecyl, n-Dodecyl, n-Tridecyl, iso-Tridecyl, n-Tetradecyl, n-Pentadecyl, n-Hexadecyl, n-Heptadecyl, n-Octadecyl oder n-Eicosyl.

Als lineare oder verzweigte Alkenylreste, die als $C_2$- bis $C_{30}$-Alkenyl- und $C_5$- bis $C_{22}$-Alkenylreste angesprochen sind, kommen z.B. in Frage: Vinyl, Allyl, Methallyl sowie die von ungesättigten Fettsäuren wie Ölsäure, Linolsäure oder Linolensäure abgeleiteten Reste mit 18 C-Atomen.

Als $C_5$- bis $C_{18}$-Cycloalkylgruppen eignen sich vor allem $C_5$- bis $C_{10}$-Cycloalkylgruppen, z.B. Cyclopentyl, Cyclohexyl, 2-, 3- oder 4-Methylcyclohexyl, 2,3-, 2,4-, 2,5- oder 2,6-Dimethylcyclohexyl, Cycloheptyl oder Cyclooctyl.

Als $C_7$- bis $C_{18}$-Aralkylgruppen kommen insbesondere gegebenenfalls alkylsubstituierte Phenylalkylgruppen mit insgesamt 7 bis 18 C-Atomen in Frage, z.B. Benzyl, 2-, 3- oder 4-Methylbenzyl, 2-Phenylethyl, 3-Phenylpropyl, 4-Phenylbutyl, 2-, 3- oder 4-Ethylbenzyl, 3- oder 4-Isopropylbenzyl oder 3- oder 4-Butylbenzyl.

Beispiele für $C_7$- bis $C_{18}$-Heteroaralkylgruppen sind Alkylreste, welche einen gegebenenfalls alkylsubstituierten fünf- oder sechsgliedrigen aromatischen Heterocyclus mit ein oder zwei Heteroatomen aus der Gruppen Stickstoff, Sauerstoff und Schwefel tragen, z.B.:

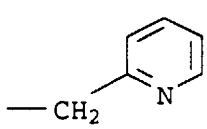 , 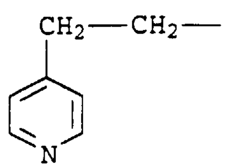 , 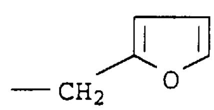 ,

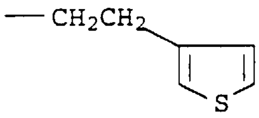 , 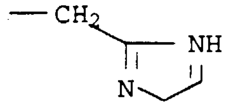 , 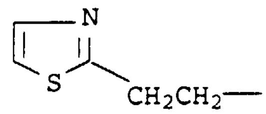 ,

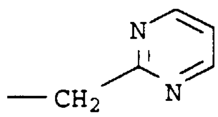 , 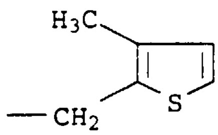 und 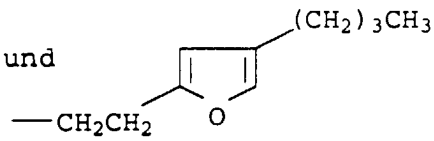

Als $C_6$- bis $C_{18}$-Arylgruppen eignen sich beispielsweise Phenyl, 2-, 3- oder 4-Bisphenyl, α- oder β-Naphthyl, 2-, 3 oder 4-Methylphenyl, 2-, 3- oder 4-Ethylphenyl, 3- oder 4-Isopropylphenyl, 3- oder 4-Butylphenyl oder 3- oder 4-(2'-Ethylhexyl)phenyl; bevorzugt werden hiervon Phenyl und $C_1$- bis $C_4$-alkylsubstituiertes Phenyl.

Als $C_6$- bis $C_{18}$-Heteroarylgruppen kommen insbesondere gegebenenfalls alkylsubstituierte fünf- oder sechsgliedrige $C_6$- bis $C_{12}$-Heteroarylgruppen mit ein oder zwei Heteroatomen aus der Gruppe Stickstoff, Sauerstoff und Schwefel in Frage, Beispiele hierfür sind:

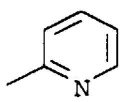 , 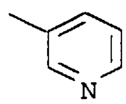 , 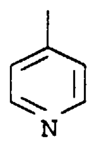 ,

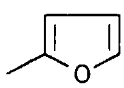 , 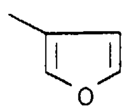 , 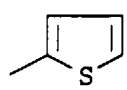 , 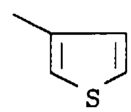 ,

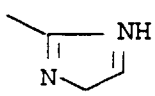 , 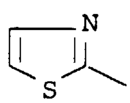 , 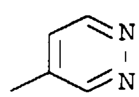 ,

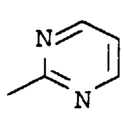 , 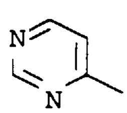 , 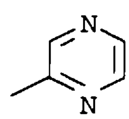 ,

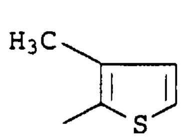 und 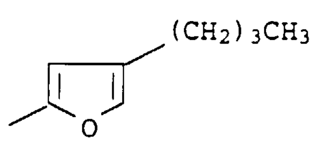

Als durch Sauerstoff oder Amino-Gruppierungen, insbesondere NH- oder $N(CH_3)$-Gruppierungen unterbrochene aliphatische Gruppierungen kommen beispielsweise folgende Reste in Betracht:

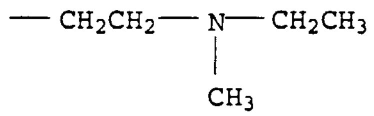 , 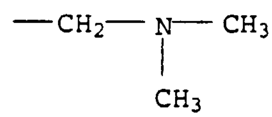 ,

$-CH_2-O-CH_3$, $-CH_2CH_2-O-CH_3$,

$-CH_2CH_2-O-CH_2CH_3$, $-CH_2CH_2-(O-CH_2CH_2)_k-H$ ,

$-(CH_2)_4-O-CH_3$, 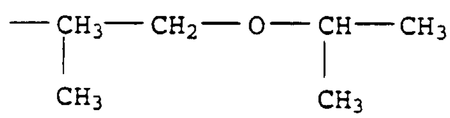

und

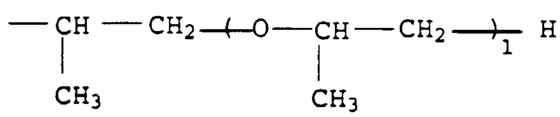

mit k = 2 bis 8 und 1 = 2 bis 5.

$C_1$- bis $C_4$-Alkoxygruppen sind insbesondere n-Propoxy, iso-Propoxy, n-Butoxy, iso-Butoxy, sec.-Butoxy, tert.-Butoxy sowie vor allem Methoxy und Ethoxy.

Als Carboxy-$C_1$- bis $C_4$-alkylgruppen kommen vorzugsweise Carboxymethyl, Carboxyethyl, Carboxypropyl, Carobxybutyl und Carboxy-tert.-butyl in Betracht.

Trägt der Phenylkern in I mehrere Reste $R^3$, können diese gleich oder verschieden sein.

In einer bevorzugten Ausführungsform setzt man N-BenzyliminiumSalze I, bei denen

$R^1$ $C_1$- bis $C_4$-Alkyl und

$R^2$ $C_5$- bis $C_{22}$-Alkyl, $C_5$- bis $C_{22}$-Alkenyl oder $C_7$- bis $C_{18}$-Phenylalkyl bedeutet, wobei aliphatische Gruppierungen zusätzlich durch ein bis drei Hydroxylgruppen, $C_1$- bis $C_4$-Alkoxygruppen, Carboxylgruppen, Carboxy-$C_1$- bis $C_4$-alkylgruppen oder Carboxyamidgruppen funktionalisiert, wobei der Phenylkern ebenfalls durch die genannten Reste substituiert sein kann, oder durch ein bis sechs nicht benachbarte Sauerstoffatome unterbrochen sein können.

Ganz besonders bevorzugt werden N-Benzyliminiumsalze I, bei denen

$R^1$ Ethyl oder insbesondere Methyl und

$R^2$ $C_8$- bis $C_{18}$-Alkyl oder $C_8$- bis $C_{12}$-Phenylalkyl bedeutet.

Unter $C_8$- bis $C_{12}$-Phenylalkylgruppen sind sowohl lineare oder verzweigte $C_2$- bis $C_6$-Alkylreste, die einen Phenylkern, vorzugsweise am Ende, tragen als auch am Phenylkern alkylsubstituierte, vorzugsweise dort ein- bis dreifach methylsubstituierte Phenylalkylreste mit insgesamt 8 bis 12 C-Atomen zu verstehen.

Hinsichtlich der Substitution am Phenylkern in I werden N-Benzyliminiumsalze I bevorzugt, bei denen

$R^3$ Wasserstoff, $C_1$- bis $C_4$-Alkyl, Hydroxyl, $C_1$- bis $C_4$-Alkoxy, Chlor, Cyano, Formyl, Carboxyl, Sulfo, Sulfato oder Carboxy-$C_1$- bis $C_4$-alkyl bezeichnet und

m für die Zahl 2 oder insbesondere 1 steht.

Als oxidationsstabile Anionen X kommen vor allem Chlorid, Bromid, Sulfat, Methosulfat, Ethosulfat, Methylsulfonat, p-Toluolsulfonat, Tetrafluoroborat, Hexafluorophosphat, Orthophosphat oder Cyanid in Betracht.

Die beschriebenen N-Benzyliminiumsalze I können am besten durch Kondensation von entsprechenden Aminen mit gegebenenfalls substituierten Benzaldehyden und anschließende Quarternierung mit alkylierenden Reagentien wie Dimethylsulfat, p-Toluolsulfonsäuremethylester oder Methylhalogenid dargestellt werden. Die allgemeine Darstellung von Iminen ist beispielsweise beschrieben in J. March, "Advanced Organic Chemistry", S. 817 (1977), übliche Quarternierungsvorschriften finden sich in Koelsch et al., J. Am. Chem. Soc. 75 (1953), S. 2095.

Es wurde gefunden, daß die beschriebenen N-Benzyliminiumsalze I besonders gute Eigenschaften als Bleichkatalysatoren zeigen, insbesondere in Textilwaschmittel-Bleichsystemen. Sie übertreffen die Wirkung von beispielsweise als Bleichkatalysatoren allgemein empfohlenen Dihydroisochinolinium-Salzen, wie sie z.B. in (1) oder (2) beschrieben werden, und zeigen in der Regel synergistische Effekte mit üblichen Peroxycarbonsäuren freisetzenden Bleichaktivatoren, insbesondere mit N,N,N',N'-Tetraacetylethylendiamin (TAED). Die Verbindungen I sind weiterhin in einfacher und kostengünstiger Weise zugänglich.

Gegenstand der vorliegenden Erfindung sind auch Wasch-, Reinigungs- und Bleichmittelformulierungen, welche ein oder mehrere N-Benzyliminiumsalze I als Bleichkatalysatoren in Mengen von 0,005 bis 15 Gew.-%, insbesondere 0,01 bis 10 Gew.-%, bezogen auf die Gesamtmenge der Formulierung enthalten.

Weiterhin sind Gegenstand der vorliegenden Erfindungen Peroxyverbindungen, insbesondere anorganische Peroxyverbindungen enthaltende Textilwaschmittelformulierungen, welche ein oder mehrere N-Benzyliminiumsalze I als Bleichkatalysatoren in Mengen von 0,01 bis 10 Gew.-%, insbesondere 0,05 bis 6 Gew.-%, vor allem 0,1 bis 3 Gew.-%, bezogen auf die Gesamtmenge der Formulierung, enthalten.

Als anorganische Peroxyverbindungen kommen Alkali. metallperborate, -percarbonate, -perphosphate, -persilicate Harnstoffperoxid, Alkylhydroperoxide und -persulfate in Betracht. Von besonderem Interesse sind Natriumperborat-Tetrahydrat, Natriumperborat-Monohydrat, Natriumpercarbonat, Cumolhydroperoxid oder tert.-Butylhydroperoxid.

Die genannten Textilwaschmittelformulierungen weisen die üblichen Zusammensetzungen auf. Hauptbestandteile sind neben den erwähnten Peroxyverbindungen, die in der Regel in Mengen von 1 bis 40 Gew.-%, insbesondere 5 bis 30 Gew.-% in den Formulierungen vorliegen, gegebenenfalls zusätzliche Bleichaktiviatoren, Gerüstsubstanzen (Builder) und Tenside. Daneben können andere übliche Hilfsstoffe und Begleitstoffe wie Vergrauungsinhibitoren, Peroxidstabilisatoren, Elektrolyte, optische Aufheller, Enzyme, Parfümöle, Schaumregulatoren und avivierende Substanzen in diesen Formulierungen vorliegen, wenn es zweckmäßig ist.

Weiterhin sind als bevorzugte Ausführungsform Gegenstand der vorliegenden Erfindung Peroxyverbindungen enthaltende Textilwaschmittelformulierungen, welche zusätzlich zu den genannten Mengen der Verbindungen I 0,1 bis 10 Gew.-%, insbesondere 0,5 bis 6 Gew.-%, bezogen auf die Gesamtmenge der Formulierung, an Peroxycarbonsäuren freisetzenden Bleichaktivatoren, insbesondere an N,N,N',N'-Tetraacetylethylendiamin (TAED) als Bleichaktivator enthalten.

Andere geeignete Bleichaktivatoren, die gegebenenfalls in Kombination mit den erfindungsgemäß verwendeten Benzyliminiumsalzen I einsetzbar sind, sind Ester, Imide, Imidazole, Oxime, Carbonsäureanhydride und Enolester, wie sie beispielsweise in den Schriften EP-A 028 432, DE-B 1246658, DE-A 3003351, WO-A 94/27970 und in der gängigen Waschmittelliteratur vielfach zitiert sind.

Die Verwendung der erfindungsgemäßen Bleichkatalysatoren zusammen mit Peroxycarbonsäuren freisetzenden Bleichaktivatoren zeigt deutliche synergistische Effekte, insbesondere bei der Bleiche hydrophober Anschmutzungen wie z.B. Chlorophyll oder Carotin. Diese synergistischen Effekte sind deutlich größer als diejenigen, die mit den in (1) und (2) als bevorzugt beschriebenen Dihydroisochinolinium-Verbindungen bei dieser Art von Anschmutzung erreicht werden. Bei der Verwendung von Kombinationen aus erfindungsgemäßen Bleichkatalysatoren und Peroxycarbonsäuren freisetzenden Bleichaktivatoren werden die Bleichkatalysatoren vorzugsweise in Mengen von 0,1 bis 3,0 Gew.-% und die Bleichaktivatoren vorzugsweise in Mengen von 0,5 bis 6 Gew.-%, jeweils bezogen auf die Gesamtmenge der Waschmittelformulierung, eingesetzt.

Gegenstand der vorliegenden Erfindung sind ebenfalls neue N-Benzyliminiumsalze der allgemeinen Formel Ia

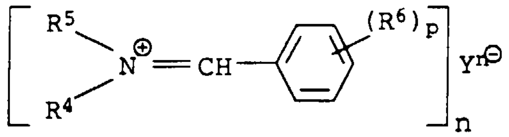

(Ia)

in der

$R^4$ $C_1$- bis $C_4$-Alkyl bezeichnet,

$R^5$ $C_5$- bis $C_{22}$-Alkyl, $C_5$- bis $C_{22}$-Alkenyl oder $C_7$- bis $C_{18}$-Phenylalkyl bedeutet, wobei aliphatische Gruppierungen zusätzlich durch ein bis drei Hydroxylgruppen, $C_1$- bis $C_4$-Alkoxygruppen, Carboxylgruppen, Carboxy-$C_1$- bis $C_4$-alkylgruppen oder Carboxyamidgruppen funktionalisiert, wobei der Phenylkern ebenfalls durch die genannten Reste substituiert sein kann, oder durch ein bis sechs nicht benachbarte Sauerstoffatome unterbrochen sein können,

$R^6$ Wasserstoff, $C_1$- bis $C_4$-Alkyl, Hydroxyl, $C_1$- bis $C_4$-Alkoxy, Chlor, Cyano, Formyl, Carboxyl, Sulfo, Sulfato oder Carboxy-$C_1$- bis $C_4$-alkyl bezeichnet,

p für die Zahl 1 oder 2 steht und

$Y^{n\ominus}$ Chlorid, Bromid, Sulfat, Methosulfat, Ethosulfat, Methylsulfonat, p-Toluolsulfonat, Tetrafluoroborat, Hexafluorophosphat, Orthophosphat oder Cyanid bezeichnet, wobei

n die Wertigkeit des Anions Y bedeutet.

Die Erfindung wird an den nachfolgenden Beispielen erläutert:

Herstellungsbeispiele

Beispiel 1

Synthese von N-(2-Phenylethyl)-N-methyl-N-benzyliminium-p-toluolsulfonat

0,5 mol (53,1 g) Benzaldehyd wurden in 80 ml Toluol vorgelegt und mit 0,5 mol (60,5 g) 2-Phenylethylamin versetzt. Der Reaktionsansatz wurde unter Rückfluß erhitzt, wobei Reaktionswasser abdestillierte. Nach ca. einer Stunde war die theoretische Menge Reaktionswasser abgeschieden und der Ansatz wurde auf Raumtemperatur abgekühlt. Anschließend versetzte man mit 0,5 mol (93,1 g) p-Toluolsulfonsäuremethylester. Der Reaktionsansatz wurde danach 3 Stunden auf 80°C erhitzt, wobei ein Niederschlag entstand. Nach Abkühlung auf Raumtemperatur wurde der Niederschlag abgesaugt und mit Toluol gewaschen. Man erhielt 141 g der Titelverbindung (entsprechend 72 % Ausbeute). Die Reinheit des Produktes lag bei > 95 %.

Beispiel 2

Synthese von N-Nonyl-N-methyl-N-benzyliminium-p-toluolsulfonat

Die Titelverbindung wurde analog zu Beispiel 1 aus n-Nonylamin, Benzaldehyd und p-Toluolsulfonsäuremethylester in einer Ausbeute von 48 % hergestellt.

Anwendungsbeispiele

Die N-Benzyliminiumsalze aus den Synthesebeispielen 1 und 2 wurden in Waschversuchen als synergistische Additive in Kombination mit bekannten, Peroxycarbonsäuren freisetzenden Bleichaktivatoren geprüft. In den Beispielen wurde als Bleichaktivator Tetraacetylethylendiamin (TAED) verwendet. Die in (1) bzw. (2) beschriebenen quatären Iminiumsalze zeigen in den gewählten typischen Textilwaschmittelformulierungen nur geringen oder keinen synergistischen Effekt bei der Kombination mit TAED. Zum Vergleich wurden Kombinationen des Iminiumsalzes A (N-Methyl-3,4-dihydroisochinolinium-methosulfat) in Kombination mit TAED geprüft.

Mit den Verbindungen aus den Synthesebeispielen 1 und 2 wurden Waschversuche mit Testanschmutzungen von Gras auf Baumwolle durchgeführt. Dabei wurde die Wirkung von erfindungsgemäß zu verwendenden Kombinationen aus Bleichaktivator (TAED) und Iminiumsalz aus Synthesebeispiel 1 bzw. 2 mit den Wirkungen von TAED allein, Iminiumsalz aus Synthesebeispiel 1 allein und Kombinationen von TAED mit Iminiumsalz A verglichen.

Die Prüfung erfolgte im Launderometer, Typ Atlas Standard, unter Verwendung eines phosphatfreien Vollwaschmittels I und eines Kompaktwaschmittels II bei folgenden Bedingungen:

| | |
|---|---|
| Flottenvolumen | 250 ml |
| Gewebe | 4 x 2,5 g verschiedene Testgewebe (mit Gras angeschmutzte gebleichte Baumwolle, Hersteller: Wäschereiforschungsanstalt Krefeld) |
| Wasserhärte | 3 mmol/l (17° dH) |
| Temperaturen | 22°, 38° und 60°C |
| Waschdauer | 30 min einschließlich Aufheizzeit |
| Spülen | 3 x 30 sec mit Leitungswasser (14° dH) |
| Waschmitteldosierung | 7,0 g/l (Waschmittel I) bzw. 4,5 g/l (Waschmittel II) |

Tabelle 1 -

| Waschmittelzusammensetzungen (Gew.-%) | | |
|---|---|---|
| Waschmittelformulierung | I | II |
| lineares Alkylbenzolsulfonat, Na-Salz | 6,25 | - |
| Fettalkoholsulfat, Na-Salz | - | 6,0 |
| $C_{13}/C_{15}$-Oxoalkohol mit 7 mol Ethylenoxid | 4,7 | 13,5 |
| Talgfettseife | 2,8 | 2,5 |
| Zeolith A | 25,0 | auf 100 |
| Natriumcarbonat | 12,0 | - |
| Natriumhydrogencarbonat | - | 6,5 |
| Natriumdisilicat | 4,5 | 0,5 |
| Magnesiumsilicat | 1,0 | - |
| Polycarboxylat (Acrylsäure/Maleinsäure Gew.-Verh. 70:30, $M_w$70000) | 3,0 | - |
| Carboxymethylcellulose (60 %) | 1,0 | 1,0 |
| Natriumperborat-Monohydrat | 10,0 | - |
| Natriumpercarbonat | - | 15,0 |
| Bleichkatalysator und/oder Bleichaktivator | s. Tab. 2-4 | s. Tab. 2-4 |
| Natriumsulfat | auf 100 | 2,0 |

Die Auswertung der Versuche wurde durch Bestimmung der Farbstärken f mittels Remissionsmessungen an den getrockneten Geweben bei 16 Wellenlängen im Bereich von 400 bis 700 nm im 20 nm-Abstand vorgenommen. Hierfür wurde aus den gemessenen Remissionswerten nach dem bei A. Kud, Seifen, Öle, Fette, Wachse 119, S. 590-594, beschriebenen Verfahren die jeweiligen Farbstärken f der Testgewebe bestimmt.

Die Bleichwirkung in % wurde daraus gemäß der nachstehenden Formel berechnet:

$$\text{Bleichwirkung [\%]} = 100 \times \frac{\text{f (vor der Wäsche) - f (nach der Wäsche)}}{\text{f (vor der Wäsche) - f (Weißgewebe vor dem Anschmutzen)}}$$

Tabelle 2 -

| Waschversuche mit Waschmittel I (Bleichwirkungen in %) | | | | |
|---|---|---|---|---|
| Beispiel Nr. | Prüfsubstanzen | 22°C | 38°C | 60°C |
| 3 | ohne Bleichkatalysator oder -aktivator | 30,6 | 40,6 | 47,8 |
| 4 | 2% TAED | 30,1 | 40,3 | 51,2 |
| 5 | 4% TAED | 31,7 | 42,5 | 49,1 |
| 6 | 6% TAED | 33,0 | 41,9 | 49,6 |
| 7 | 0,1 % Synthesebeispiel 1 | 30,0 | 40,0 | 48,3 |
| 8 | 0,5 % Synthesebeispiel 1 | 31,9 | 40,0 | 49,2 |
| 9 | 2,5 % Synthesebeispiel 1 | 31,4 | 41,2 | 51,3 |
| 10 | 4 % TAED + 0,1 % Synthesebeispiel 1 | 35,6 | 48,0 | 53,6 |
| 11 | 4 % TAED + 0,5 % Synthesebeispiel 1 | 40,5 | 52,2 | 58,0 |
| 12 | 4 % TAED + 2,5 % Synthesebeispiel 1 | 43,7 | 53,5 | 58,3 |

Tabelle 3 -

| Waschversuche mit Waschmittel II (Bleichwirkungen in %) | | | | |
|---|---|---|---|---|
| Beispiel Nr. | Prüfsubstanzen | 22°C | 38°C | 60°C |
| 13 | ohne Bleichkatalysator oder -aktivator | 23,8 | 27,3 | 36,1 |
| 14 | 5 % TAED | 27,0 | 34,0 | 43,4 |
| 15 | 5 % TAED + 0,5 % Iminiumsalz A | 26,3 | 33,2 | 43,8 |
| 16 | 5 % TAED + 0,5 % Synthesebeispiel 1 | 36,1 | 42,9 | 49,5 |
| 17 | 5 % TAED + 0,5 % Synthesebeispiel 2 | 37,5 | 45,3 | 52,6 |

Tabelle 4 -

| Waschversuche mit Waschmittel II (Bleichwirkungen in %) | | | | |
|---|---|---|---|---|
| Beispiel Nr. | Prüfsubstanzen | 22°C | 38°C | 60°C |
| 18 | ohne Bleichkatalysator oder -aktivator | 22,8 | 31,7 | 42,8 |
| 19 | 5 % TAED | 25,5 | 35,8 | 43,4 |
| 20 | 5 % TAED + 0,10 % Iminiumsalz A | 27,0 | 34,6 | 43,5 |
| 21 | 5 % TAED + 0,25 % Iminiumsalz A | 28,5 | 34,4 | 44,4 |
| 22 | 5 % TAED + 0,50 % Iminiumsalz A | 29,5 | 36,5 | 46,2 |
| 23 | 5 % TAED + 0,10 % Synthesebeispiel 1 | 28,0 | 37,7 | 46,6 |
| 24 | 5 % TAED + 0,25 % Synthesebeispiel 1 | 30,1 | 40,7 | 46,7 |
| 25 | 5 % TAED + 0,50 % Synthesebeispiel 1 | 33,8 | 43,2 | 49,8 |

Die Ergebnisse in Tabelle 2 zeigen, daß die erfindungsgemäß in Kombination mit TAED zu verwendenden N-Benzyliminiumsalze I besser wirksam sind als die Verbindungen I oder TAED alleine bzw. die rechnerische Summe der Einzelwirkungen. Die Ergebnisse in Tabelle 2 bis 4 zeigen, daß die erfindungsgemäß in Kombination mit TAED zu verwendenden Verbindungen I wesentlich besser wirksam sind als Kombinationen von TAED mit Bleichkatalysatoren vom Typ wie sie in (1) bzw. (2) beschrieben sind.

**Patentansprüche**

1. Verwendung von N-Benzyliminiumsalzen der allgemeinen Formel I

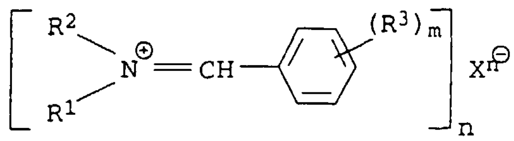

(I)

in der

$R^1$ und $R^2$ unabhängig voneinander Wasserstoff, $C_1$- bis $C_{30}$-Alkyl, $C_2$- bis $C_{30}$-Alkenyl, $C_5$- bis $C_{18}$-Cycloalkyl, $C_7$- bis $C_{18}$-Aralkyl oder -Heteroaralkyl oder $C_6$- bis $C_{18}$-Aryl oder -Heteroaryl bedeuten, wobei aliphatische Gruppierungen zusätzlich durch ein bis fünf Hydroxylgruppen, $C_1$- bis $C_4$-Alkoxygruppen, Aminogruppen, $C_1$- bis $C_4$-Alkylaminogruppen, Di-$C_1$- bis $C_4$-alkylaminogruppen, Chloratome, Bromatome, Nitrogruppen, Cyanogruppen, $C_1$- bis $C_4$-Thioalkylgruppen, $C_1$- bis $C_4$-Sulfoalkylgruppen, Carboxylgruppen, Sulfogruppen, Carboxy-$C_1$- bis $C_4$-alkylgruppen, Carboxyamidgruppen oder Phenyl-, Tolyl- oder Benzylreste funktionalisiert, wobei aromatische, cycloaliphatische und heterocyclische Struktureinheiten ebenfalls durch die genannten Reste substituiert sein können, oder durch ein bis acht nicht benachbarte Sauerstoffatome, Aminogruppen, $C_1$- bis $C_4$-Alkylaminogruppen oder Carbonylgruppen unterbrochen sein können,
mit der Maßgabe, daß wenigstens einer der Reste $R^1$ oder $R^2$ mindestens 5 C-Atome im Falle einer aliphatischen Struktur von $R^1$ bzw. $R^2$ oder mindestens 7 C-Atome im Falle des Vorliegens eines cyclischen Strukturelementes in $R^1$ bzw. $R^2$ aufweist,

$R^3$ die unter $R^1$ bzw. $R^2$ aufgeführten Bedeutungen oder Hydroxyl, Mercapto, $C_1$- bis $C_4$-Alkoxy, Amino, $C_1$- bis $C_4$-Alkylamino, Di-$C_1$- bis $C_4$-alkylamino, Chlor, Brom, Nitro, Cyano, $C_1$- bis $C_4$-Thioalkyl, $C_1$- bis $C_4$-Sulfoalkyl, Formyl, Carboxyl, Sulfo, Sulfato, Carboxy-$C_1$- bis $C_4$-alkyl, Carboxamid, Phenyloxy, Tolyloxy oder Benzyloxy bezeichnet,

m für die Zahl 1, 2 oder 3 steht und

$X^{n\ominus}$ ein oxidationsstabiles Anion bezeichnet, wobei

n die Wertigkeit des Anions X bedeutet,

als Bleichkatalysatoren.

2. Verwendung von N-Benzyliminiumsalzen I gemäß Anspruch 1 als Bleichkatalysatoren in Textilwaschmittel-Bleichsystemen.

3. Verwendung nach Anspruch 1 oder 2 von N-Benzyliminiumsalzen I, bei denen

$R^1$ $C_1$- bis $C_4$-Alkyl und

$R^2$ $C_5$- bis $C_{22}$-Alkyl, $C_5$- bis $C_{22}$-Alkenyl oder $C_7$- bis $C_{18}$-Phenylalkyl bedeutet, wobei aliphatische Gruppierungen zusätzlich durch ein bis drei Hydroxylgruppen, $C_1$- bis $C_4$-Alkoxygruppen, Carboxylgruppen, Carboxy-$C_1$- bis $C_4$-alkylgruppen oder Carboxyamidgruppen funktionalisiert, wobei der Phenylkern ebenfalls durch die genannten Reste substituiert sein kann, oder durch ein bis sechs nicht benachbarte Sauerstoffatome unterbrochen sein können.

4. Verwendung nach Anspruch 1 oder 2 von N-Benzyliminiumsalzen I, bei denen

$R^1$ Ethyl oder Methyl und

$R^2$ $C_8$- bis $C_{18}$-Alkyl oder $C_8$- bis $C_{12}$-Phenylalkyl bedeutet.

**5.** Verwendung nach Anspruch 1 oder 2 von N-Benzyliminiumsalzen I, bei denen

$R^3$ Wasserstoff, $C_1$- bis $C_4$-Alkyl, Hydroxyl, $C_1$- bis $C_4$-Alkoxy, Chlor, Cyano, Formyl, Carboxyl, Sulfo, Sulfato oder Carboxy-$C_1$- bis $C_4$-alkyl bezeichnet und

m für die Zahl 1 oder 2 steht.

**6.** Verwendung nach Anspruch 1 oder 2 von N-Benzyliminiumsalzen I, bei denen X Chlorid, Bromid, Sulfat, Methosulfat, Ethosulfat, Methylsulfonat, p-Toluolsulfonat, Tetrafluoroborat, Hexafluorophosphat, Orthophosphat oder Cyanid bezeichnet.

**7.** Wasch-, Reinigungs- und Bleichmittelformulierungen, enthaltend ein oder mehrere N-Benzyliminiumsalze I gemäß den Ansprüchen 1 bis 6 als Bleichkatalysatoren in Mengen von 0,005 bis 15 Gew.-%, bezogen auf die Gesamtmenge der Formulierung.

**8.** Textilwaschmittelformulierungen, enthaltend Peroxyverbindungen aus der Gruppe Alkalimetallperborate, -percarbonate, -perphosphate, -persilicate, -persulfate, Harnstoffperoxid und Alkylhydroperoxide in Mengen von 1 bis 40 Gew.-% sowie ein oder mehrere N-Benzyliminiumsalze I gemäß den Ansprüchen 1 bis 6 als Bleichkatalysatoren in Mengen von 0,01 bis 10 Gew.-%, jeweils bezogen auf die Gesamtmenge der Formulierung.

**9.** Textilwaschmittelformulierungen nach Anspruch 8, enthaltend zusätzlich 0,1 bis 10 Gew.-%, bezogen auf die Gesamtmenge der Formulierung, an Peroxycarbonsäuren freisetzenden Bleichaktivatoren.

**10.** Textilwaschmittelformulierungen nach Anspruch 8, enthaltend zusätzlich 0,1 bis 10 Gew.-%, bezogen auf die Gesamtmenge der Formulierung, an N,N,N',N'-Tetraacetylethylendiamin als Bleichaktivator.

**11.** N-Benzyliminiumsalze der allgemeinen Formel Ia

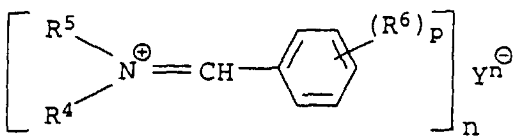

(Ia)

in der

$R^4$ $C_1$- bis $C_4$-Alkyl bezeichnet,

$R^5$ $C_5$- bis $C_{22}$-Alkyl, $C_7$- bis $C_{18}$-Phenylalkyl oder von Ölsäure, Linolsäure oder Linolensäure abgeleitete Reste bedeutet, wobei aliphatische Gruppierungen zusätzlich durch ein bis drei Hydroxylgruppen, $C_1$- bis $C_4$-Alkoxygruppen, Carboxylgruppen, Carboxy-$C_1$- bis $C_4$-alkylgruppen oder Carboxyamidgruppen funktionalisiert, wobei der Phenylkern ebenfalls durch die genannten Reste substituiert sein kann, oder durch ein bis sechs nicht benachbarte Sauerstoffatome unterbrochen sein können,

$R^6$ Wasserstoff, $C_1$- bis $C_4$-Alkyl, Hydroxyl, $C_1$- bis $C_4$-Alkoxy, Chlor, Cyano, Formyl, Carboxyl, Sulfo, Sulfato oder Carboxy-$C_1$- bis $C_4$-alkyl bezeichnet,

p für die Zahl 1 oder 2 steht und

$Y^{n\ominus}$ Chlorid, Bromid, Sulfat, Methosulfat, Ethosulfat, Methylsulfonat, p-Toluolsulfonat, Tetrafluoroborat, Hexafluorophosphat, Orthophosphat oder Cyanid bezeichnet, wobei

n die Wertigkeit des Anions Y bedeutet.

## Claims

**1.** The use of N-benzyliminium salts of the general formula I

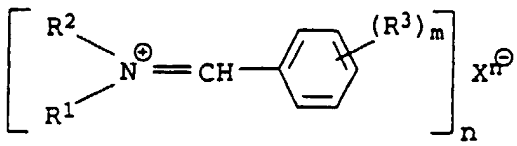

(I)

where

| | |
|---|---|
| $R^1$ and $R^2$ are | independently hydrogen, $C_1$-$C_{30}$-alkyl, $C_2$-$C_{30}$-alkenyl, $C_5$-$C_{18}$-cycloalkyl, $C_7$-$C_{28}$-aralkyl or -heteroaralkyl or $C_6$-$C_{18}$-arkyl or -heteroaryl, wherein aliphatic groupings may additionally be functionalized by one to five hydroxyl groups, $C_1$-$C_4$-alkoxy groups, amino groups, $C_1$-$C_4$-alkylamino groups, di-$C_1$-$C_4$-alkylamino groups, chlorine atoms, bromine atoms, nitro groups, cyano groups, $C_1$-$C_4$-thioalkyl groups, $C_1$-$C_4$-sulfoalkyl groups, carboxyl groups, sulfo groups, carboxy-$C_1$-$C_4$-alkyl groups, carboxyamide groups or phenyl, tolyl or benzyl radicals (wherein aromatic, cycloaliphatic and heterocyclic structural units may likewise be substituted by the radicals mentioned) or interrupted by one to eight nonadjacent oxygen atoms, amino groups, $C_1$-$C_4$-alkylamino groups or carbonyl groups,<br>with the proviso that at least one of $R^1$ and $R^2$ has at least 5 carbon atoms in the event of $R^1$ or $R^2$, as the case may be, having an aliphatic structure or at least 7 carbon atoms in the event of a cyclic structural element being present in $R^1$ or $R^2$, as the case may be, |
| $R^3$ | has the meanings recited for $R^1$ and $R^2$ or is hydroxyl, mercapto, $C_1$-$C_4$-alkoxy, amino, $C_1$-$C_4$-alkylamino, di- $C_1$-$C_4$-alkylamino, chlorine, bromine, nitro, cyano, $C_1$-$C_4$-thioalkyl, $C_1$-C-sulfoalkyl, formyl, carboxyl, sulfo, sulfato, carboxy-$C_1$-$C_4$-alkyl, carboxamide, phenyloxy, tolyloxy or benzyloxy, |
| m | is 1, 2 or 3, and |
| $X^{n\ominus}$ | is an oxidation-stable anion, wherein |
| n | is the valence of the anion X, |

as bleach catalysts.

**2.** The use of N-benzyliminium salts I as per claim 1 as bleach catalysts in textile detergent bleaching systems.

**3.** The use as claimed in claim 1 or 2 of N-benzyliminium salts I where

$R^1$ is $C_1$-$C_4$-alkyl, and
$R^2$ is $C_5$-$C_{22}$-alkyl, $C_5$-$C_2$-alkenyl or $C_7$-$C_{18}$-phenylalkyl, wherein aliphatic groupings may additionally be functionalized by one to three hydroxyl groups, $C_1$-$C_4$-alkoxy groups, carboxyl groups, carboxy-$C_1$-$C_4$-alkyl groups or carboxyamide groups (wherein the phenyl nucleus may likewise be substituted by the radicals mentioned) or interrupted by one to six nonadjacent oxygen atoms.

**4.** The use as claimed in claim 1 or 2 of N-benzyliminium salts I

$R^1$ is ethyl or methyl and
$R^2$ is $C_8$-$C_{18}$-alkyl or $C_8$-$C_{12}$-phenylalkyl.

**5.** The use as claimed in claim 1 or 2 of N-benzyliminium salts I where

$R^3$ is hydrogen, $C_1$-$C_4$-alkyl, hydroxyl, $C_1$-$C_4$-alkoxy, chlorine, cyano, formyl, carboxyl, sulfo, sulfato or carboxy-$C_1$-$C_4$-alkyl, and
m is 1 or 2.

**6.** The use as claimed in claim 1 or 2 of N-benzyliminium salts I where X is chloride, bromide, sulfate, methosulfate,

ethosulfate, methylsulfonate, p-toluenesulfonate, tetrafluoroborate, hexafluorophosphate, orthophosphate or cyanide.

**7.** Washing, cleaning and bleaching agent formulations containing one or more N-benzyliminium salts I as per claims 1 to 6 as bleach catalysts in amounts of 0.005 to 15% by weight, based on the total amount of the formulation.

**8.** Textile detergent formulations containing peroxy compounds selected from alkali metal perborates, percarbonates, perphosphates, persilicates, persulfates, urea peroxide and alkyl hydroxyperoxides in amounts of 1 to 40% by weight and also one or more N-benzyliminium salts I as per claims 1 to 6 as bleach catalysts in amounts of 0.01 to 10% by weight, each based on the total amount of the formulation.

**9.** Textile detergent formulations as claimed in claim 8, additionally containing 0.1 to 10% by weight, based on the total amount of the formulation, of bleach activators which release peroxycarboxylic acids.

**10.** Textile detergent formulations as claimed in claim 8, additionally containing 0.1 to 10% by weight, based on the total amount of the formulation, of N,N,N',N'-tetraacetylethylenediamine as bleach activator.

**11.** N-Benzyliminium salts of the general formula Ia

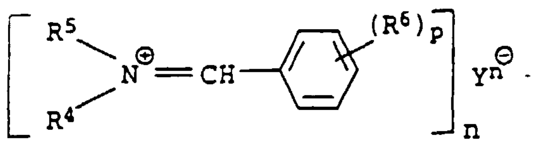

(Ia)

where

$R^4$ is $C_1$-$C_4$-alkyl,
$R^5$ is $C_5$-$C_{22}$-alkyl, $C_7$-$C_{18}$-Phenylalkyl or radicals derived from oleic acid, linoleic acid or linolenic acid, wherein aliphatic groupings may additionally be functionalized by one to three hydroxyl groups, $C_1$-$C_4$-alkoxy groups, carboxyl groups, carboxy-$C_1$-$C_4$-alkyl groups or carboxyamide groups (wherein the phenyl nucleus may likewise be substituted by the radicals mentioned) or interrupted by one to six nonadjacent oxygen atoms,
$R^6$ is hydrogen, $C_1$-$C_4$-alkyl, hydroxyl, $C_1$-$C_4$-alkoxy, chlorine, cyano, formyl, carboxyl, sulfo, sulfato or carboxy-$C_1$-$C_4$-alkyl,
p is 1 or 2, and
$Y^{n\ominus}$ is chloride, bromide, sulfate, methosulfate, ethosulfate, methylsulfonate, p-toluenesulfonate, tetrafluoroborate, hexafluorophosphate, orthophosphate or cyanide, wherein
n is the valence of the anion Y.

**Revendications**

**1.** Utilisation de sels de N-benzyliminium de formule générale I

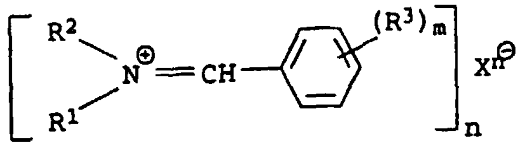

(I)

dans laquelle

$R^1$ et $R^2$ représentent indépendamment l'un de l'autre, un atome d'hydrogène, un groupement alkyle en $C_1$-$C_{30}$, alcényle en $C_2$-$C_{30}$, cycloalkyle en $C_5$-$C_{18}$, aralkyle en $C_7$-$C_{18}$ ou hétéroaralkyle en $C_7$-$C_{18}$ ou aryle en $C_6$-$C_{18}$ ou hétéroaryle en $C_6$-$C_{18}$, où les groupements aliphatiques peuvent en outre être

fonctionnalisés par un à cinq groupements hydroxyle, des groupements alcoxy en $C_1$-$C_4$, des groupements amino, des groupements (alkyle en $C_1$-$C_4$)amino, des groupements di(alkyle en $C_1$-$C_4$)amino, des atomes de chlore, de brome, des groupements nitro, cyano, des groupements thioalkyle en $C_1$-$C_4$, des groupements sulfoalkyle en $C_1$-$C_4$, des groupements carboxyle, sulfo, des groupements carboxy(alkyle en $C_1$-$C_4$), des groupements carboxamides ou des restes phényle, tolyle, ou benzyle, où les unités structurelles aromatiques, cycloaliphatiques et hétérocycliques peuvent éventuellement être substituées par les restes cités, ou peuvent être interrompus par un à huit atomes d'oxygène non voisins, par des groupements amino, par des groupements (alkyle en $C_1$-$C_4$)amino ou par des groupements carbonyle,

étant spécifié que l'un au moins des restes $R^1$ ou $R^2$ présente au moins 5 atomes de carbone si $R^1$ respectivement $R^2$ a une structure aliphatique ou bien au moins 7 atomes de carbone si $R^1$ respectivement $R^2$ a un élément structurel cyclique,

$R^3$ prend les significations données pour $R^1$ respectivement $R^2$ ou représente des groupements hydroxyle, mercapto, alcoxy en $C_1$-$C_4$, amino, (alkyle en $C_1$-$C_4$)amino, di(alkyle en $C_1$-$C_4$)amino, chloro, bromo, nitro, cyano, thioalkyle en $C_1$-$C_4$, sulfoalkyle en $C_1$-$C_4$, formyle, carboxyle, sulfo, sulfato, carboxy (alkyle en $C_1$-$C_4$), carboxamide, phényloxy, tolyloxy ou benzyloxy,

m est mis pour le nombre 1, 2 ou 3 et

$X^{n-}$ représente un anion stable à l'oxydation, où

n représente la valence de l'anion X,

en tant que catalyseur de blanchiment.

**2.** Utilisation de sels de N-benzyliminium I selon la revendication 1 en tant que catalyseur de blanchiment dans des systèmes de blanchiment d'agents de lavage pour textiles.

**3.** Utilisation selon la revendication 1 ou 2 de sels de N-benzyliminium I, dans lesquels

$R^1$ représente un groupement alkyle en $C_1$-$C_4$, et

$R^2$ représente un groupement alkyle en $C_5$-$C_{22}$, alcényle en $C_5$-$C_{22}$ ou phénylalkyle en $C_7$-$C_{18}$, où les groupements aliphatiques peuvent en outre être fonctionnalisés par un à trois groupements hydroxyle, des groupements alcoxy en $C_1$-$C_4$, des groupements carboxyle, des groupements carboxy(alkyle en $C_1$-$C_4$) ou des groupements carboxamides, où le noyau phénylique peut éventuellement être substitué par les restes cités, ou peuvent être interrompus par un à six atomes d'oxygène non voisins.

**4.** Utilisation selon la revendication 1 ou 2 de sels de N-benzyliminium I, dans lesquels

$R^1$ représente un groupement éthyle ou méthyle, et

$R^2$ représente un groupement alkyle en $C_8$-$C_{18}$ ou phénylalkyle en $C_8$-$C_{12}$.

**5.** Utilisation selon la revendication 1 ou 2 de sels de N-benzyliminium I, dans lesquels

$R^3$ représente un atome d'hydrogène, des groupements alkyle en $C_1$-$C_4$, hydroxyle, alcoxy en $C_1$-$C_4$, chloro, cyano, formyle, carboxyle, sulfo, sulfato ou carboxy(alkyle en $C_1$-$C_4$), et

m est mis pour le nombre 1 ou 2.

**6.** Utilisation selon la revendication 1 ou 2 de sels de N-benzyliminium I, dans lesquels X représente un groupement chlorure, bromure, sulfate, méthosulfate, éthosulfate, méthylsulfonate, p-toluènesulfonate, tétrafluoroborate, hexafluorophosphate, orthophosphate ou cyanure.

**7.** Formulations d'agents de lavage, de nettoyage et de blanchiment contenant un ou plusieurs sels de N-benzyliminium I selon l'une quelconque des revendications 1 à 6, en tant que catalyseurs de blanchiment dans des quantités de 0,005 à 15% en poids, par rapport à la quantité totale de formulation.

**8.** Formulations d'agent de lavage pour textiles contenant des composés peroxy choisis dans le groupe des perborates de métaux alcalins, des percarbonates de métaux alcalins, des perphosphates de métaux alcalins, des persilicates de métaux alcalins, des persulfates de métaux alcalins, des peroxydes d'urée et des hydroperoxydes d'alkyle, dans des quantités de 1 à 40% en poids, ainsi qu'un ou plusieurs sels de N-benzyliminium I selon l'une quelconque des revendications 1 à 6, en tant que catalyseur de blanchiment dans des quantités de 0,01 à 10%

en poids, à chaque fois par rapport à la quantité totale de formulation.

**9.** Formulations d'agent de lavage pour textiles selon la revendication 8, contenant en outre 0,01 à 10% en poids, par rapport à la quantité totale de formulation, d'activateurs de blanchiment libérant des acides peroxycarboxyliques.

**10.** Formulations d'agent de lavage pour textiles selon la revendication 8, contenant en outre 0,01 à 10% en poids, par rapport à la quantité totale de formulation, de N,N,N',N'-tétraacétyléthylènediamine en tant qu'activateurs de blanchiment.

**11.** Sels de N-benzyliminium de formule générale la

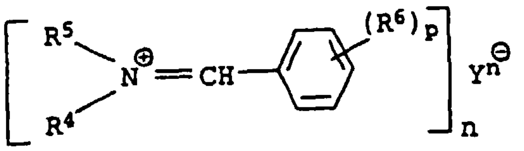

(Ia)

dans laquelle

$R^4$ représente un groupement alkyle en $C_1$-$C_4$, et
$R^5$ représente un groupement alkyle en $C_5$-$C_{22}$, phénylalkyle en $C_7$-$C_{18}$ ou des restes dérivant de l'acide oléique, de l'acide linoléique ou de l'acide linolénique, où les groupements aliphatiques peuvent en outre être fonctionnalisés par un à trois groupements hydroxyle, des groupements alcoxy en $C_1$-$C_4$, des groupements carboxyle, des groupements carboxy(alkyle en $C_1$-$C_4$) ou des groupements carboxamides, où le noyau phénylique peut éventuellement être substitué par les restes cités, ou peuvent être interrompus par un à six atomes d'oxygène non voisins,
$R^6$ représente un atome d'hydrogène, des groupements alkyle en $C_1$-$C_4$, hydroxyle, alcoxy en $C_1$-$C_4$, chloro, cyano, formyle, carboxyle, sulfo, sulfato ou carboxy(alkyle en $C_1$-$C_4$),
p est mis pour le nombre 1 ou 2 et
$Y^{n\ominus}$ représente un groupement chlorure, bromure, sulfate, méthosulfate, éthosulfate, méthylsulfonate, p-toluènesulfonate, tétrafluoroborate, hexafluorophosphate, orthophosphate ou cyanure, où
n représente la valence de l'anion Y.